# EUROPEAN PATENT APPLICATION

(11) **EP 2 545 895 A2**
(43) Date of publication of application: **16.01.2013**
(21) Application number: 12175879.1
(22) Date of filing: 11.07.2012
(51) Int. Cl.: A61K 6/04

(54) **Palladium-Cobalt based alloys**

(30) Priority: 12.07.2011 US 201113181172
(71) Applicant: Ivoclar Vivadent, Inc., Amherst, NY 14228 (US)
(72) Inventor: Dasgupta, Tridib, East Amherst, NY New York 14051 (US); Tysowsky, George, East Amherst, NY New York 14051 (US)
(74) Representative: UEXKÜLL & STOLBERG

(57) **Abstract**

A nonmagnetic alloy is provided based on a palladium - cobalt binary system with the addition of gold, has a coefficient of thermal expansion (CTE) of about 13.8 to about 15 and may include one or more of the following additive metals: aluminum, boron, chromium, gallium, lithium, rhenium, ruthenium, silicon, tantalum, titanium, and tungsten.

## Description

### Cross-Reference to Related Applications

This application is a continuation in part application of U.S. Application No. 11/892,933, filed August 28, 2007, which claims priority pursuant to 35 USC §119 to Provisional Application No. 60/844,672, filed September 15, 2006, all of which are incorporated herein by reference.

### Field of the Invention

This invention provides a novel palladium-cobalt based alloy. The alloy can be used, for example, in making cast metal dental articles or restorations and, in particular, for alloy-porcelain (porcelain fused to metal ("PFM)) restorations.

### Background of the Invention

In the discussion that follows, reference is made to certain structures and/or methods. However, the following references should not be construed as an admission that these structures and/or methods constitute prior art. Applicant expressly reserves the right to demonstrate that such structures and/or methods do not qualify as prior art.

Since the late 1950s, dental crowns, bridges, and the like have been made with a composite including a cast metal substrate with a veneer of porcelain fabricated in such a manner that there is a bond between metal and porcelain such that the composite is stronger than the individual component parts. There are several aspects to be addressed when formulating such composites.

Aesthetics is one aspect to be considered. The primary reason for the use of such a composite is to reproduce the normal coloration of natural dentition. The enamel layer of healthy natural dentition is quite translucent and porcelain can be made with equal translucency. The translucency of enamel allows the color of healthy dentine to be seen. The dentine color normally has a yellowish tint. For a porcelain/alloy combination to be effective as a composite, a layer of oxide must be present on the alloy to form a bond with the porcelain. While high gold alloys may provide a suitable yellowish background for the porcelain for proper aesthetics, the alloying elements can form a dark gray to black colored oxide layer, which can screen out this underlying yellowish background color. Moreover, larger amounts of alloying elements form a colored oxide layer that can further reduce or eliminate the underlying gold color of the alloy.

Mechanical properties are another aspect to be considered. The American National Standards Institute/American Dental Association ("ANSI/ADA") specification #38 and International Organization for Standardization ("ISO") standard IS09693 require a yield strength of at least 250 megapascal ("MPa") for the alloy. To attain such strength in gold-based alloys, significant amounts of alloying elements must be added, the result being alloys having a color that is closer to gray. It was thought that it is necessary to provide great strength because the alloy supported porcelain, which had little strength, particularly in tension, and zero ductility. Any slight deformation of the metal can cause fracture of the porcelain layer. The minimum for the standards mentioned above were set on the basis of testing alloys that were being successfully used at the time of the development of the standards. Subsequently, the minimum requirement has been questioned since alloys with less than this minimum have been used successfully. Also, it has been shown that the minimum requirement for single crowns should be lower than that for crowns composed of three or more unit bridges.

An unpublished work at the University of Kiel in Germany has indicated that from 30 to 35 kilograms of force causes pain to patients while, in one instance, 75 kilograms of force caused fracture of the tooth.

Physical properties are another aspect to be considered. Although the abovementioned standards do not require either minimum or maximum values for the coefficient of thermal expansion ("CTE"), these standards require that the CTE value be given for both porcelain and alloy. This is because the popular conception is that the coefficients of porcelain and metal should be "matched" in order to assure compatibility of the two. This concept fails to take into consideration that stresses between the two occur during cooling rather than during heating, and the cooling rates of porcelain and metal vary very significantly.

It is readily understood that the solidus of the alloy must be sufficiently higher than the firing temperature of the porcelain so that the alloy is not even partially melted during firing of the porcelain.

Chemical properties are another aspect to be considered. The bonding of porcelain to metal does not occur directly; rather it occurs between porcelain and a metal oxide layer. Normal PFM procedure is to heat the cast alloy to a suitable temperature to produce a metal oxide layer on the surface of the alloy. If this oxide does not adhere to the alloy, it can be simply removed by its attachment to the porcelain. Some of the bond is simply mechanical but the primary bonding takes place as a mutual solution of metal oxide in porcelain and vice versa known as diffusion bonding. If the oxide is not soluble in the porcelain and/or vice versa, no bonding takes place. When the porcelain is fired, small particles and larger particle surfaces are fused (melted) and this liquid porcelain and the metal oxide layer form a solution by either liquid or solid diffusion.

In general, it is desirable that all alloys for dental applications are nonmagnetic. It was found that certain dental alloys create a magnetic effect that is not desirable in dental applications. It would be beneficial to provide a dental alloy with good corrosion resistance and having non-magnetic properties.

### Summary

The present invention provides compositions, materials and techniques that can optionally address one or more of the abovementioned shortcomings associated with conventional technology.

An aspect of the present invention provides an alloy which can be manufactured by the normal melt process, cast into a bar and rolled to the required thickness or alternatively, by the atomization and compression method of U.S. Pat. No. 5,799,386 to Ingersoll et al. entitled Process Of Making Metal Castings, issued Sep. 1, 1998, which is incorporated herein by reference in its entirety.

Another aspect of the present invention provides an alloy which has a solidus high enough that no fusion occurs during firing of normal porcelains.

Another aspect of the present invention provides an alloy which has a CTE in a range that has been shown to be compatible with porcelains.

Another aspect of the present invention is to provide an alloy which can be readily cast by normal dental procedures, and can be recast using normal dental laboratory procedures.

Another aspect of the present invention provides a cast alloy unit which can be ground and polished to a high shine.

Another aspect of the present invention provides an alloy which has a light oxide color that does not affect the apparent color of the porcelain layer and the oxide does not increase during the firing of the porcelain.

Another aspect of the present invention provides an alloy which when heated to the porcelain firing temperature, a thin, continuous, tenacious oxide is formed and creates a strong bond between the alloy and the porcelain by diffusion bonding.

Another aspect of the present invention provides an alloy which has the strength to withstand loads in excess of those that would cause pain to the patient.

An alloy formed according to one embodiment of the invention is a palladium - cobalt binary alloy wherein palladium is about 20 to about 90 wt.% and cobalt is about 10 to about 80 wt.%. The coefficient of thermal expansion (CTE) is about 14.0 to about 15.3. From about 0 wt. % up to about 20 wt.% of the following metals can be added to the base Pd/Co alloy: aluminum, boron, chromium, gallium, lithium, rhenium, ruthenium, silicon, tantalum, titanium, tungsten or combinations thereof.

Another aspect of the present invention is to provide an alloy comprising about 27 to about 30 wt.% Pd, about 55 to about 58 wt.% Co, about 8 to about 11 wt.% Cr, about 2.5 to about 4 wt.% W, about 1 to about 2.5 wt.% Ga and less than about 1 wt.% Al, Si, B, Li, or combinations thereof.

Another aspect of the present invention is to provide a dental article or restoration comprising a dental porcelain composition fused to a dental alloy, the alloy comprising from about 20 to about 90 wt.% Pd, about 10 to about 80 wt.% Co and about 0 to about 20 wt.% aluminum, boron, chromium, gallium, lithium, rhenium, ruthenium, silicon, tantalum, titanium, tungsten or combinations thereof.

According to yet another aspect, the present invention provides an alloy comprising a base composition comprising palladium and cobalt, the amount of palladium and cobalt, expressed as a ratio is about 10:80 to about 80:10, the alloy further comprising about 0 to about 30 wt.% additives selected from: Au, Pt, Cr, Mo, W, Fe, AI, Si, Mn, Ga, Ta, Ti, Ru, Re, and combinations thereof, wherein the coefficient of thermal expansion of the alloy is about 14.0 to about 15.5 at 25-500°C.

According to an additional aspect, the present invention provides one or more of the above-described alloys in combination with a ceramic or a glass-ceramic material, which can optionally comprise porcelain.

According to further aspects, the alloy(s) and ceramic or glass-ceramic are bonded together, optionally by an oxide layer. According to still further aspects, a dental article, such as, for example, a restoration such as a crown or a bridge can comprise the alloy and/or combination of the present invention.

According to yet another aspect, an embodiment of the present invention provides a palladium cobalt alloy further including gold. The alloy exhibits non-magnetic properties and good corrosion resistance. The alloy may further comprise additional additives selected from Pt, Cr, Mo, W, Fe, AI, Si, Mn, Ga, Ta, Ti, Ru, Re, and combinations thereof

These and other aspects of the present invention will become apparent upon a review of the following detailed description and accompanying examples which are recited herein as illustrative of the present invention but in no way limit the present invention.

### Brief Description Of The Drawings

Figure 1 is a schematic illustration of a dental article formed according to one aspect of the present invention.

Figure 2 is a magnified sectional view of a portion the article of Figure 1.

### Detailed Description

There are several properties exhibited by alloy(s) of the present invention that make it suitable for porcelain fused to metal (PFM) applications. The alloy is grey in color with an oxide coating for bonding porcelain to the oxidized cast alloy substrate. The alloy has mechanical properties for cast prostheses and for the support of the porcelain and is readily polished to a bright sheen. The alloy is based on a portion of the palladium - cobalt binary system wherein palladium is about 20 to about 90 wt.% and cobalt is about 10 to about 80 wt.% to obtain a coefficient of thermal expansion (CTE) in the range of about 14.0 to about 15.3. Up to about 20 wt% of the following metals can be added to the base Pd/Co alloy: aluminum, boron, chromium, gallium, lithium, rhenium, ruthenium, silicon, tantalum, titanium, tungsten or combinations thereof, to improve physical, chemical, mechanical and handling properties. The alloy of the invention can have a solidus high enough that no melting occurs during firing of normal porcelains, and a coefficient (CTE) in a range that has been demonstrated to be compatible with porcelains.

The alloy of the invention can be readily cast by normal dental procedures, and can be recast using normal dental laboratory procedures. The cast alloy unit can be ground and polished to a high shine. The alloy can have a light oxide color that does not affect the apparent color of the porcelain layer and the oxide does not increase during the firing of the porcelain. When heated to the porcelain firing temperature, a thin, continuous, tenacious oxide is formed, which enters into a bond with the porcelain. The alloy has a strength that withstands loads in excess of those that would cause pain to the patient.

The alloy of the present invention can meet aesthetic needs while using a palladium-cobalt base. That is, the alloy system reproduces the normal coloration of natural dentition. The enamel layer of healthy natural dentition is quite translucent and porcelain can be made with similar translucency. The translucency of enamel allows the color of healthy dentine to be seen. This color normally has a yellowish tint. With the porcelain alloy combination, a layer of oxide must be present to form a bond with the porcelain. While high gold alloys may provide a yellowish background for the porcelain, other metals they are cost prohibitive and alloys such as nickel, cobalt, palladium, etc., provide a gray background.

For proper bonding, the alloying elements form an oxide on the cast metal surface. This dark gray to black colored oxide layer, can affect the apparent color of the porcelain veneering layer. The alloy system of the present invention may include elements added to regulate the amount and color of the oxide layer, selected from the group including, but not limited to: aluminum, boron, chromium, silicon, iron, manganese, and/or gallium.

The mechanical properties of the alloy follow ANSI/ADA specification #38 and ISO standard IS09693 which require yield strength of at least about 250 MPa for the alloy. To attain such strength, significant amounts of alloying elements selected from the group comprising, but not limited to: chromium, silicon, tantalum, titanium, and/or tungsten may be added to the alloy formulation.

The above mentioned standards do not require minimum or maximum values for coefficient of thermal expansion (CTE); however, physical properties including the CTE value for both porcelain and alloy may be regulated. The alloy of the invention may include elements added to regulate the grain size, selected from the group including, but not limited to: chromium, gallium, tantalum, titanium, tungsten, rhenium and/or ruthenium. Elements that can be added to regulate oxidation during melting and casting include but are not limited to: aluminum, boron, lithium, silicon, iron, manganese, and/or gallium. Also, heat transfer rate may be taken into consideration. When cooling from the porcelain firing temperature, shrinkage of both porcelain and alloy take place and the alloy, which cools faster, shrinks faster and thus puts tensile forces on the porcelain to metal bond. If this disparity of shrinkage is too much, the porcelain will no longer be bonded to the alloy when the composite reaches room temperature. It is readily understood that the solidus of the alloy must be sufficiently higher than the firing temperature of the porcelain so that the alloy is not even partially melted during firing. Concerning the bonding of the porcelain to the alloy of the invention, it does not occur between porcelain and metal, it occurs between porcelain and the metal oxide layer formed when the alloy is heated prior to and during the firing of the porcelain. If the oxide is not adherent to the alloy, it can be simply removed by the porcelain. Some of the bond is simply mechanical but the primary bonding takes place as diffusion of metal oxide in porcelain and vice versa. If the oxides are not soluble in the porcelain and/or vice versa, no bond takes place.

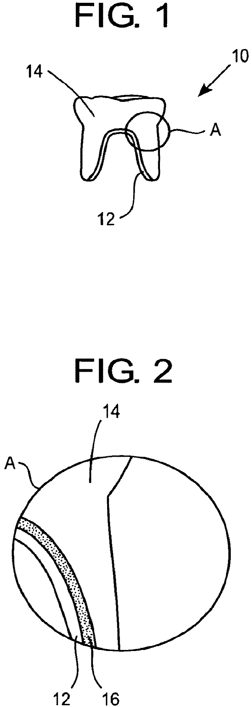

An illustrative embodiment of certain aspects of the present invention is shown in Figures 1-2. A composite comprising an alloy formed according to the present invention is illustrated therein. Specifically, the composite is illustrated in the form of a dental article 10, such as a restoration. As illustrated, the dental article 10 may include an alloy 12 formed according to the principles of the present invention, as set forth above, in combination with a second material, such as a porcelain layer 14 bonded thereto. As best illustrated in Figure 2, the alloy 12 is bonded to the second material or porcelain 14 via an oxide layer 16, as described herein. Of course, the present invention is not limited to the illustrated embodiment, and numerous alternative composites and/or dental articles are contemplated.

In another embodiment of the present invention, it is preferable that the palladium-cobalt alloy includes the addition of gold. The inclusion of gold to the alloy improves the corrosion resistance of the alloy and improves fluidity of the product during casting. The alloy may further comprise additional additives selected from Pt, Cr, Mo, W, Fe, AI, Si, Mn, Ga, Ta, Ti, Ru, Re, and combinations thereof.

For certain alloys herein, the solidus point, which is defined by the starting of the melting range, is at least about 1050°C in order to accommodate the use of high fusing porcelains in the market without any distortion of the dental prosthesis. It further allows repair work using pre-solders available in the market with no damage to the restoration. For certain alloys herein, the liquidus point, which is defined by the end of the melting range is not greater than about 1350°C for easy melting of the alloy. For most of the induction casting machines on the market, the maximum temperature limit is 1500°C. In order to melt the metal and reach the proper fluidity for casting, at least 100° C above the liquidus point of the alloy must be reached.

It is preferable that the composition comprises the following components:

**Table 1**

| Element | Weight Percent Range 1 | Weight Percent Range 2 | Weight Percent Range 3 | Weight Percent Range 4 |
|---|---|---|---|---|
| Gold | 0 - about 5 % | about 0.40 to about 4% | about 1 to about 3% | about 0.4 to about 3% |
| Palladium | about 20 - about 36.7 % | about 24.5 to about 35% | about 24.5 to about 35% | about 24.5 to about 30% |
| Cobalt | about 38 - about 54% | about 38 - about 50% | about 38 - about 50% | about 38 - about 44 % |
| Chromium | 16 - about 25 % | about 17 - about 22 % | about 18 - about 22% | about 18 - about 22 % |
| Molybdenum | 0 - about 12.9 % | about 9 - about 12.9 % | about 10 - about 12.9 % | about 10 - about 12.9 % |
| Tungsten | 0 - about 13 % | about 1.5 - about 13 % | about 1.5 - about 13% | about 1.5 - about 4 % |
| Gallium | 0 - about 2.6 % | about 1.0 - about 2.6% | about 2.0 - about 2.6 % | about 2.0 - about 2.6 % |
| Aluminum | 0 - about 3.4 % | 0 - about 3.4 % | 0 - about 3.4 % | 0 - about 3.4 % |
| Silicon | 0 - about 0.5 % | 0 - about 0.5 % | 0 - about 0.5 % | 0 - about 0.5 % |
| Ruthenium | 0 - about 0.5 % | 0 - about 0.5 % | 0 - about 0.5 % | 0 - about 0.5 % |
| Rhenium | 0 - about 0.5 % | 0 - about 0.5 % | 0 - about 0.5 % | 0 - about 0.5 % |
| Manganese | 0 - about 0.3 % | 0 - about 0.3 % | 0 - about 0.3 % | 0 - about 0.3 % |
| Iron | 0 - about 0.3 % | 0 - about 0.3 % | 0 - about 0.3 % | 0 - about 0.3 % |
| Boron | 0 - about 0.2 % | 0 - about 0.2 % | 0 - about 0.2 % | 0 - about 0.2 % |
| Lithium | 0 - about 0.2 % | 0 - about 0.2 % | 0 - about 0.2 % | 0 - about 0.2 % |

The C.T.E @ 25 - 500°C of the alloy should be between about 13.9 - about 14.9 x 10⁻⁶ /°C /unit length in order to work with most of the high fusing porcelains on the market.

The iron in the composition increases the bond strength of the alloy to the ceramic. The gallium and the boron in the composition aid in controlling the liquidus temperature and improves the melting and casting properties. The chromium in the composition improves the corrosion resistance and promotes the non-magnetic property. The molybdenum and tungsten in the composition assist in controlling the CTE, more specifically, in reducing the CTE.

The combination of Au, Al, Si, Ga, B, and Li reduces the temperature, improves the fluidity of the molten alloy and controls the CTE. The combination of Au, Cr, and Pd increases the corrosion resistance, controls the CTE and improves biocompatibility. The combination of Fe and Mn improves the bond strength of the metal-ceramic system. The combination of Mo and W controls the CTE by reducing the number in the range of about 13.9 - about 14.9 x 10-6/°C / unit length @ 25 - 500 °C. It is preferable that the combination of Au and Pd are equal to or greater than 25.0%. The combination of Au and Pd of greater than or equal to 25.0 % qualifies the alloy as a noble dental alloy. It is preferable that Au and Pd are the only noble metals present in the composition. The combination of Au having a melting temperature of 1065°C and Pd having a melting temperature of 1554°C provides an optimal melting temperature for the dental alloy, compatible with many dental porcelains.

Moreover, most of the compositions set forth in Table 1 above are nonmagnetic and all of the compositions in Ranges 2 and 3 are nonmagnetic. It is important to add the correct amount of Pd and Co in the composition to provide nonmagnetic properties. Too much Co can produce a magnetic alloy. Co must be balanced with Pd to provide nonmagnetic properties. The amount of Cr also influences the magnetic properties, helping to demagnetize the composition if a certain amount of Co is present. When the presence of Co is more than 11 % of the Pd and Co total, the amount of Cr must be at least 16% to provide a nonmagnetic alloy.
In the following further embodiments of the invention are disclosed by means of numbered paragraphs.
1. A dental alloy comprising a base composition comprising the following in weight percent:

| | |
|---|---|
| palladium | about 20 to about 36.7 |
| cobalt | about 38 to about 54 |
| chromium | about 16 to about 22 |
| gold | about 0.1 to about 5 |
| aluminum | 0 to about 3.4 |
| molybdenum | 0 to about 12.9 |
| tungsten | 0 to about 13 |
| gallium | 0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2 |
| wherein the dental alloy is nonmagnetic | |
| wherein molybdenum, tungsten or the combination thereof is 3% or greater. | |

2. The dental alloy of paragraph 1 having a coefficient of thermal expansion is from about 13.8 to about 15.0 at 25 - 500°C.
3. The dental alloy of paragraph 1 wherein the amount of palladium and gold is 25% or greater.
4. The dental alloy of paragraph 1 wherein palladium and gold are the only noble metals in the composition.
5. The dental alloy of paragraph 1 wherein the melting temperature is in the range of about 1040 C to about 1350 C.
6. In combination, the alloy of paragraph 1 bonded to a ceramic or glass-ceramic material.
7. The combination of paragraph 6, wherein the ceramic or glass-ceramic material comprises porcelain.
8. The combination of paragraph 6, wherein the bond comprises an oxide layer.
9. A dental article comprising the combination ofparagraph 6.
10. The dental article ofparagraph 9, comprising: a dental crown or dental bridge.
11. A dental alloy comprising a base composition comprising the following in weight percent:

| | |
|---|---|
| palladium | about 24.5 to about 35 |
| cobalt | about 38 to about 50 |
| chromium | about 17 to about 22 |
| gold | about 0.1 to about 5 |
| aluminum | 0 to about 3.4 |
| molybdenum | about 9 to about 12.9 |
| tungsten | about 1.5 to about 13 |
| gallium | about 1.0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2 |
| wherein the dental alloy is nonmagnetic. | |

12. The dental alloy of paragraph 11 wherein the amount of palladium and gold is 25% or greater.
13. The dental alloy of paragraph 11 wherein palladium and gold are the only noble metals in the composition.
14. The dental alloy of paragraph 11 wherein the melting temperature is in the range of about 1040 C to about 1350 C.
15. In combination, the alloy of paragraph 11 bonded to a ceramic or glass-ceramic material.
16. The combination of paragraph 15, wherein the ceramic or glass-ceramic material comprises porcelain.
17. The combination of paragraph 15, wherein the bond comprises an oxide layer.
18. A dental article comprising the combination of paragraph 15.
19. The dental article of paragraph 18, comprising: a dental crown or dental bridge.
20. A dental alloy comprising a base composition comprising the following in weight percent:

| | |
|---|---|
| palladium | about 24.5 to about 35 |
| cobalt | about 38 to about 50 |
| chromium | about 18 to about 22 |
| gold | about 1 to about 3 |
| aluminum | 0 to about 3.4 |
| molybdenum | about 10 to about 12.9 |
| tungsten | about 1.5 to about 13 |
| gallium | about 2.0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2 |
| wherein the dental alloy is nonmagnetic. | |

21. The dental alloy of paragraph 20 wherein the amount of palladium and gold is 25% or greater.
22. The dental alloy of paragraph 20 wherein palladium and gold are the only noble metals in the composition.
23. The dental alloy of paragraph 20 wherein the melting temperature is in the range of about 1040 C to about 1350 C.
24. In combination, the alloy of paragraph 20 bonded to a ceramic or glass-ceramic material.
25. The combination of paragraph 24, wherein the ceramic or glass-ceramic material comprises porcelain.
26. The combination of paragraph 24, wherein the bond comprises an oxide layer.
27. A dental article comprising the combination of paragraph 24.
28. The dental article of paragraph 27, comprising: a dental crown or dental bridge.
29. A dental alloy comprising a base composition comprising the following in weight percent:

| | |
|---|---|
| palladium | about 24.5 to about 30 |
| cobalt | about 38 to about 44 |
| chromium | about 18 to about 22 |
| gold | about 0.4 to about 3 |
| aluminum | 0 to about 2.0 |
| molybdenum | about 10 to about 12.9 |
| tungsten | about 1.5 to about 4 |
| gallium | about 2.0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2 |
| wherein the dental alloy is nonmagnetic. | |

30. The dental alloy of paragraph 29 wherein the amount of palladium and gold is 25% or greater.
31. The dental alloy of paragraph 29 wherein palladium and gold are the only noble metals in the composition.
32. The dental alloy of paragraph 29 wherein the melting temperature is in the range of about 1040 C to about 1350 C.
33. In combination, the alloy of paragraph 29 bonded to a ceramic or glass-ceramic material.
34. The combination of paragraph 33, wherein the ceramic or glass-ceramic material comprises porcelain.
35. The combination of paragraph 33, wherein the bond comprises an oxide layer.
36. A dental article comprising the combination of paragraph 33.
37. The dental article of paragraph 36, comprising: a dental crown or dental bridge.

The following examples are for the purpose of illustration. It is understood that such detail is solely for that purpose, and variations can be made therein by those skilled in the art without departing from the spirit and scope of the invention which is defined by the following claims.

Examples 1 — 7 (Coefficient of thermal expansion (CTE))

For successful use of the alloys of the invention with porcelains in contemporary use, the CTE should be in the range of about 14.0 to about 15.3. When two metals comprise the base of an alloy, it would be expected that the CTE of such an alloy be somewhere between the CTE's of each metals. It has been determined that this does not necessarily hold necessarily true for alloys of palladium and cobalt. Whereas Pd has a CTE of 12.5 and Co 11.75, the alloys of the invention comprising an alloy of Pd/Co have higher values as shown in the following examples, where the amounts listed are in % by weight:

**Table 2**

| Ex. No. | 1 | 2 | 3 | 4 | 5 | 6 | 7 |
|---|---|---|---|---|---|---|---|
| Pd | 10 | 20 | 30 | 40 | 50 | 70 | 90 |
| Co | 90 | 80 | 70 | 60 | 50 | 30 | 10 |
| CTE | 13.85 | 14.0 | 14.1 | 14.6 | 14.9 | 15.2 | 14.2 |

EXAMPLES 8 - 12 (Solidus)

The minimum solidus temperature of alloys of certain embodiments of the invention is determined to be about 1025°C, more preferably 1040°C, in order that the alloy does not start to melt during the firing of porcelain on its surface.

**Table 3**

| Ex. No. | 8 | 9 | 10 | 11 | 12 |
|---|---|---|---|---|---|
| Pd | 65 | 33.8 | 61.8 | 27.0 | 28.2 |
| Co | 35 | 60.4 | 31.0 | 52.3 | 56.0 |
| Cr | | | 1.2 | 16.2 | 10.0 |
| Mo | | 2.4 | 2.0 | | |
| Si | | 1.0 | 0.7 | 0.6 | 0.05 |
| Fe | | | | 0.3 | |
| W | | | | | 3.0 |
| Ga | | | | | 2.0 |
| Al | | 1.2 | 1.6 | | 0.35 |
| Ta | | | 0.8 | | |
| Nb | | | | 3.0 | |
| Re | | 0.6 | | | |
| Ru | | 0.6 | 0.8 | 0.5 | |
| Li | | | 0.1 | 0.1 | 0.2 |
| B | | | | | 0.2 |
| Solidus T | 1219°C | 1014°C | 1250°C | 976°C | 1047°C |

Alloys 9 and 11 do not appear to meet the required minimum solidus temperature. Example 13 is set forth in Table 4 below.

**Table 4**

| |
|---|
| Example 13: TYPE: Noble PFM / Type-4 / ISO 9693 31- VI |
| Composition: Palladium: 28+/- 0 .80 %; Co: 55 - 58 %; Cr: 8.0 - 11.0 %; W: 2.5 - 4.0 %; Ga: 1.0 - 2.5 %; (Al, Si, B & Li: < 1.0 %). |
| Density: 9.0gm/cc |
| Color: Crucible: WHITE Ceramic |
| Bum out Temperature: 750 - 820°C (1380° - 1510°F) |
| Casting Temperature: 1410 - 1460°C (2570 - 2660°F) |
| Melting Temperature: 1100 - 1350°C (2010 - 2460°F) |
| Oxidation Cycle: 925°C / 5 minute / AIR |
| Porcelain Compatibility: IPS d. Sign; IPS Classics & InLine. |
| Tensile Properties: (after stimulated porcelain firing cycle) |
| U.T.S: 800 MPa |
| 0.2 % offset Proof Stress: 610 MPa Percent Elongation 9.0% |
| Mod. Of Elasticity: 175,000 MPa |
| Hardness: 365 VHN |
| C.T.E: @ 25-500°C @ 20 - 600°C :14.2 x 10⁻⁶/°C/inch/inch; 14.8 x10⁻⁶/°C/inch /inch |

Examples 14-21 (Solidus) are set forth in Table 5 below:

**Table 5**

| Elements | 14 | 15 | 16 | 17 | 18 | 19 | 20 | 21 |
|---|---|---|---|---|---|---|---|---|
| Pd | 23 | 21.1 | 23 | 29.3 | 36.3 | 22 | 16.2 | 12.5 |
| Co | 46.6 | 58.6 | 54 | 58 | 48 | 50 | 50.5 | 58.5 |
| Cr | 24.4 | 16.1 | 16 | | | 14.5 | 18.2 | 11.5 |
| Au | 2.2 | 3.7 | 1.8 | 6.0 | 9.0 | | | |
| Pt | | | | | | 4.0 | 5.8 | 9.0 |
| Mo | | | | 1.0 | 0.9 | 1.5 | 3.0 | 3.0 |
| W | 3.4 | | 3.5 | | | | | |
| Mn | 0.1 | 0.1 | 0.1 | | | 0.5 | .6 | 0.5 |
| Al | | | | 3.0 | 3.4 | | | |
| Si | | | | 1.2 | 1.0 | 0.8 | 0.7 | 1.0 |
| Ga | | | 1.2 | | | 1.5 | | |
| B | 0.1 | 0.1 | 0.1 | | | | | |
| Ti | | | | | 1.2 | | | |
| Ta | | | | 0.6 | 0.5 | | 1.0 | |
| Re | 0.1 | 0.2 | 0.2 | | | | | |
| Ru | | | | 0.8 | 0.8 | 3.0 | 4.0 | 4.0 |
| Li | 0.1 | 0.1 | 0.1 | 0.1 | 0.1 | | | |
| Solidus: | 1182 °C | 1133° C | 1088° C | 955° C | 1013 °C | 1010° C | 1243 °C | 1197° C |

Alloys 17, 18 and 19 do not meet the required minimum solidus temperature.

Examples 22 through 44

The following examples show melting properties :

**Table 6**

| Exp # | 22* | 23* | 24* | 25* | 26* | 27* | 28* |
|---|---|---|---|---|---|---|---|
| Au | 5 | 5 | 3 | X | X | X | X |
| Pd | 35.2 | 35.8 | 36.7 | 29.8 | 31.7 | 25.1 | 26 |
| Co | 53 | 52 | 54 | 51.6 | 51 | 50 | 39 |
| Cr | X | X | X | 18.8 | 18 | 19 | 22 |
| Mo | 0.5 | X | X | X | X | X | X |
| W | X | 0.7 | X | X | X | 3 | 10.2 |
| Ga | 1.6 | 1.8 | 1.6 | 2 | 1.5 | 2.6 | 2.1 |
| Al | 3.3 | 3.4 | 3.2 | 0.5 | 0.5 | X | 0.35 |
| Si | 0.4 | 0.3 | 0.5 | X | X | X | 0.05 |
| Ru | 0.5 | 0.5 | 0.5 | X | 0.5 | X | 0.1 |
| Re | X | X | X | 0.5 | X | 0.2 | X |
| Mn | 0.3 | 0.3 | 0.3 | X | X | X | X |
| Fe | X | X | X | 0.2 | 0.2 | X | X |
| B | 0.15 | 0.15 | 0.15 | 0.2 | 0.2 | 0.1 | X |
| Li | 0.05 | 0.05 | 0.05 | X | X | 0.1 | 0.2 |
| Ta | X | X | X | X | X | X | X |
| Melting Range | 991 - 1330 C | 827 - 1351 C | 1096- 1344 C | 1045 - 1334 C | 1058 - 1370 C | 1114 - 1351 C | 1101 - 1327 C |
| CTE @ 500C | 13.4 | 13.4 | 13.9 | 15.5 | | 15.3 | 15.2 |

| | | | | | | | |
|---|---|---|---|---|---|---|---|
| *Control | | | | | | | |

**Table 7**

| Exp # | 29 | 30 | 31 | 32 | 33 | 34* | 35 |
|---|---|---|---|---|---|---|---|
| Au | 2 | 1.4 | 1.6 | 0.8 | 0.8 | X | 0.6 |
| Pd | 23.4 | 23.8 | 23.6 | 24.6 | 24.6 | 25.2 | 24.8 |
| Co | 39 | 42 | 39 | 39.4 | 39.4 | 38 | 39.4 |
| Cr | 20 | 20 | 20 | 20.4 | 20.4 | 21.4 | 20.4 |
| Mo | X | X | 12.9 | 11.6 | X | 12 | 11.8 |
| W | 12.7 | 11 | X | X | 11.6 | X | X |
| Ga | 2 | 1.1 | 2.2 | 2 | 2 | 2.2 | 2 |
| Al | 0.55 | 0.35 | 0.35 | 0.7 | 0.7 | 0.7 | 0.55 |
| Si | 0.05 | 0.05 | 0.05 | 0.1 | 0.1 | 0.1 | 0.05 |
| Ru | 0.1 | 0.1 | 0.1 | X | X | X | X |
| Re | X | X | X | X | X | X | X |
| Re | X | X | X | X | X | X | X |
| Mn | X | X | X | X | X | X | X |
| Fe | X | X | X | X | X | X | X |
| B | X | X | X | 0.1 | 0.1 | 0.1 | 0.2 |
| Li | 0.2 | 0.2 | 0.2 | 0.1 | 0.1 | 0.1 | 0.2 |
| Ta | X | X | X | X | X | X | X |
| Melting Range | 1062 - 1334 C | 1051 - 1323 C | 1078 - 1262 C | 1025 - 1259 C | 1198 - 1312 C | 1153 - 1262 C | 1053 - 1260 C |
| CTE @ 500C | 14.4 | 14.7 | 14 | 14.2 | 14.4 | 14 | 14.2 |

| | | | | | | | |
|---|---|---|---|---|---|---|---|
| *Control | | | | | | | |

**Table 8**

| Exp # | 36* | 37* | 38* | 39 | 40 | 41 | 42 | 43 | 44 |
|---|---|---|---|---|---|---|---|---|---|
| Au | X | 1.5 | 1.5 | 0.4 | 0.5 | 0.5 | 0.5 | 0.6 | 0.5 |
| Pd | 25.3 | 24.05 | 24.05 | 24.75 | 24.65 | 24.6 | 24.6 | 24.5 | 24.6 |
| Co | 38.2 | 51.1 | 51.1 | 40 | 38.5 | 39 | 39 | 38.8 | 38.9 |
| Cr | 21 | 17 | 17 | 21.4 | 21 | 21 | 21 | 21.2 | 21 |
| Mo | 12 | 2.8 | 1.8 | 12.7 | 10.5 | 10.5 | 10.4 | 10.5 | 10.5 |
| W | X | X | 1 | 0.5 | 2.5 | 2 | 2 | 2 | 2 |
| Ga | 2.5 | 2 | 2 | X | 2.1 | 2.1 | 2.1 | 1.8 | 2.1 |
| Al | 0.55 | X | X | X | X | X | X | X | X |
| Si | 0.05 | X | X | X | X | X | X | X | X |
| Ru | X | X | X | X | X | X | X | X | X |
| Re | X | 1.0 | 1.0 | X | X | X | X | 0.1 | X |
| Mn | X | X | X | X | X | X | X | X | X |
| Fe | X | X | X | 0.2 | 0.2 | 0.2 | 0.2 | 0.3 | 0.2 |
| B | 0.2 | 0.05 | 0.05 | X | X | 0.1 | 0.15 | 0.2 | 0.2 |
| Li | 0.2 | X | X | 0.05 | 0.05 | X | 0.05 | X | X |
| Ta | X | 0.5 | 0.5 | X | X | X | X | X | X |
| Melting Range | 1100-1275 C | 1055 - 1342 C | 1040 - 1350 C | 1224 - 1283 | 1073 - 1284 C | 1090 - 1284 | 1090 - 1280 | 1078 - 1270 C | 1050-1278 C |
| | | | | C | | C | C | | |
| CTE @ 500C | 14.2 | 15.06 | 15.23 | 14.7 | 14.2 | 14.1 | 14.3 | 14.26 | 14.37 |

| | | | | | | | | | |
|---|---|---|---|---|---|---|---|---|---|
| *Control | | | | | | | | | |

The control examples are outside the range of the preferred composition. Examples 22- 24 are magnetic. The rest of the Examples are non-magnetic. Examples 25- 28 and 36 do not contain Au and either exhibit a high CTE or are difficult to melt. Examples 37 and 38 do not contain the requisite amount of Mo or W and are outside the required CTE.

It is preferable that the CTE is in the range of about 13. 8 to 15 at 25 - 500°C.

All numbers expressing quantities of ingredients, constituents, reaction conditions, and so forth used in the specification are to be understood as being modified in all instances by the term "about." Notwithstanding that the numerical ranges and parameters set forth, the broad scope of the subject matter presented herein are approximations, the numerical values set forth are indicated as precisely as possible. Any numerical value, however, may inherently contain certain errors resulting, for example, from their respective measurement techniques, as evidenced by standard deviations therefrom.

Although the present invention has been described in connection with preferred embodiments thereof, it will be appreciated by those skilled in the art that additions, deletions, modifications, and substitutions not specifically described may be made without departing from the spirit and scope of the invention as defined in the appended claims.

## Claims

1. A dental alloy comprising a base composition comprising the following in weight percent:
| | |
|---|---|
| palladium | about 20 to about 36.7 |
| cobalt | about 38 to about 54 |
| chromium | about 16 to about 22 |
| gold | about 0.1 to about 5 |
| aluminum | 0 to about 3.4 |
| molybdenum | 0 to about 12.9 |
| tungsten | 0 to about 13 |
| gallium | 0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2, |
| wherein the dental alloy is nonmagnetic, and | |
| wherein molybdenum, tungsten or the combination thereof is 3% or greater. | |

2. The dental alloy of claim 1 having a coefficient of thermal expansion from about 13.8 to about 15.0 at 25 - 500°C.

3. The dental alloy of claim 1, wherein the amount of palladium and gold is 25% or greater.

4. The dental alloy of claim 1, wherein palladium and gold are the only noble metals in the composition.

5. The dental alloy of claim 1, wherein the melting temperature is in the range of about 1040 C to about 1350 C.

6. A dental alloy according to any one of claims 1 to 5 comprising a base composition comprising the following in weight percent:
| | |
|---|---|
| palladium | about 24.5 to about 35 |
| cobalt | about 38 to about 50 |
| chromium | about 17 to about 22 |
| gold | about 0.1 to about 5 |
| aluminum | 0 to about 3.4 |
| molybdenum | about 9 to about 12.9 |
| tungsten | about 1.5 to about 13 |
| gallium | about 1.0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2. |

7. A dental alloy according to any one of claims 1 to 5 comprising a base composition comprising the following in weight percent:
| | |
|---|---|
| palladium | about 24.5 to about 35 |
| cobalt | about 38 to about 50 |
| chromium | about 18 to about 22 |
| gold | about 1 to about 3 |
| aluminum | 0 to about 3.4 |
| molybdenum | about 10 to about 12.9 |
| tungsten | about 1.5 to about 13 |
| gallium | about 2.0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2. |

8. A dental alloy according to any one of claims 1 to 5 comprising a base composition comprising the following in weight percent:
| | |
|---|---|
| palladium | about 24.5 to about 30 |
| cobalt | about 38 to about 44 |
| chromium | about 18 to about 22 |
| gold | about 0.4 to about 3 |
| aluminum | 0 to about 2.0 |
| molybdenum | about 10 to about 12.9 |
| tungsten | about 1.5 to about 4 |
| gallium | about 2.0 to about 2.6 |
| silicon | 0 to about 0.5 |
| ruthenium | 0 to about 0.5 |
| rhenium | 0 to about 0.5 |
| manganese | 0 to about 0.3 |
| boron | 0 to about 0.2 |
| iron | 0 to about 0.3, |
| lithium | 0 to about 0.2. |

9. The dental alloy according to any one of claims 1 to 8 bonded to a ceramic or glass-ceramic material.

10. The dental alloy of claim 9, wherein the ceramic or glass-ceramic material comprises porcelain.

11. The dental alloy of claim 9, wherein the bond comprises an oxide layer.

12. A dental article comprising the dental alloy according to any one of claims 9 to 11.

13. The dental article of claim 12, comprising: a dental crown or dental bridge.
